# EUROPEAN PATENT SPECIFICATION

(11) **EP 2 822 595 B1**
(45) Date of publication and mention of the grant of the patent: **23.05.2018**
(21) Application number: 13722577.7
(22) Date of filing: 06.03.2013
(51) Int. Cl.: A61K 31/137, A61K 39/39, A61K 45/06, A61P 37/04, A61K 31/133, A61K 31/381, A61K 31/4245, A61K 31/437, A61K 31/661

(54) **SPHINGOSINE-1 -PHOSPHATE RECEPTORS MODULATORS FOR IMPROVING VACCINE IMMUNIZATION**
SPHINGOSIN-1-PHOSPHATREZEPTORMODULATOREN ZUR VERBESSERUNG DER IMPFSTOFFIMMUNISIERUNG
MODULATEURS DES RÉCEPTEURS DE LA SPHINGOSINE-1-PHOSPHATE POUR AMÉLIORER L'IMMUNISATION VACCINALE

(30) Priority: 07.03.2012 IT RM20120083
(43) Date of publication of application: 14.01.2015
(73) Proprietor: Istituto Superiore di Sanità, 00161 Roma (IT)
(72) Inventor: VENDETTI, Silvia, 00161 Roma (IT)
(74) Representative: Gitto, Serena
(86) International application number: PCT/IT2013/000070
(87) International publication number: WO 2013/132526

(56) References cited:
- WO-A1-2008/124210
- WO-A2-2007/003904
- SANTUCCI M B ET AL: "Sphingosine 1-phosphate promotes antigen processing and presentation to CD4+ T cells in Mycobacterium tuberculosis-infected monocytes", BIOCHEMICAL AND BIOPHYSICAL RESEARCH COMMUNICATIONS, vol. 361, no. 3, 28 September 2007 (2007-09-28), pages 687-693, XP022624909, ACADEMIC PRESS INC. ORLANDO, FL, US ISSN: 0006-291X, DOI: 10.1016/J.BBRC.2007.07.087 [retrieved on 2007-08-11]
- MAEDA YASUHIRO ET AL: "Sphingosine 1-phosphate receptor type 1 regulates egress of mature T cells from mouse bone marrow.", INTERNATIONAL IMMUNOLOGY, vol. 22, no. 6, June 2010 (2010-06), pages 515-525, XP002699334, ISSN: 1460-2377
- CIABATTINI ANNALISA ET AL: "Distribution of Primed T Cells and Antigen-Loaded Antigen Presenting Cells Following Intranasal Immunization in Mice", PLOS ONE, vol. 6, no. 4, E19346, April 2011 (2011-04), pages 1-8, XP002699335, ISSN: 1932-6203
- MARC BIGAUD ET AL: "Second generation S1P pathway modulators: Research strategies and clinical developments", BIOCHIMICA ET BIOPHYSICA ACTA (BBA) - MOLECULAR AND CELL BIOLOGY OF LIPIDS, vol. 1841, no. 5, 1 May 2014 (2014-05-01), pages 745-758, XP055384137, AMSTERDAM, NL ISSN: 1388-1981, DOI: 10.1016/j.bbalip.2013.11.001
- Mary Premenko-Lanier ET AL: "Transient FTY720 treatment promotes immune-mediated clearance of a chronic viral infection", Nature, vol. 454, no. 7206, 14 August 2008 (2008-08-14), pages 894-898, XP055384139, ISSN: 0028-0836, DOI: 10.1038/nature07199

## Description

The present invention refers to the use of sphingosine 1-phosphate receptor modulators for improving vaccine immunizations. Particularly, the invention refers to the use of sphingosine 1-phosphate receptor modulators, as FTY720 compound, by means of administration combined with vaccines in order to improve vaccine immunizations and therefore reducing doses and increasing vaccines effectiveness.

Vaccines represent one of the main means of medicine to fight infections. Vaccination is to simulate as much as possible a natural infection providing protection from future pathogen infections. In spite of the obtained results, great effort in order to develop effective vaccines for preventing illness against which, to date, each vaccine strategy have been ineffective, or against new and emerging diseases, is still necessary. Being largely formed by purified molecules, the majority of used currently or developing vaccines are not able to stimulate an effective immune response, as it happens during natural infection. Therefore, it is necessary the addition of adjuvant substances or immune-agonists in order vaccines to be effective. The most part of vaccines currently used are derived from an empirical fight infection approach. In fact, vaccines prepared using attenuated germs, anatoxins, subunits of microorganisms, microbial products are provided. The development of technologies and the progress of immune system knowledge allow new vaccines to be developed or already used vaccines to be reformulated, in order to reduce doses to a minimum and therefore reducing related side effects. Among the most common vaccines, smallpox vaccine, antirabic vaccine, antitetanus vaccine, polio vaccine, flu vaccine, hepatitis vaccine may be cited and many others used both in developed country and in developing country where several diseases are endemic.

In the light of above, it is therefore apparent the need to provide new vaccine therapy able to overcome known vaccines disadvantages.

The identification of regulatory mechanisms of immune responses which control the amplification and the regulation thereof after immunization, are important and useful for the discovery of specific immunological targets to be used in order to obtain effective vaccinations.

It is known that the generation of adaptive immune responses against potential pathogens, allergens, tumors or also "self" antigens take place in the lymph nodes. The quality of CD4⁺ e CD8⁺ lymphocytes "priming" is critical in order to induce an effective immunological memory in the protection against infections or diseases onset (22).
Dendritic cells (DCs) catch antigens in tissues and migrate to draining lymph nodes after maturation, where they present antigenic peptides on their surface to T lymphocytes by means of major histocompatibility complex molecules (MHC I and II) (3, 21). Naive T lymphocytes pass trough the peripheral blood and lymphatic system, go into lymph nodes and return into circulation when they do not meet the antigen (4). The beginning of immune response needs the presence and the contact between antigen presenting cells, in particular activated DCs, and antigen-specific T-lymphocytes in the area of secondary lymphoid organs (7). T lymphocytes specific for an antigen are in the T lymphocyte repertories of each individual in the form of a low number of precursors. Said cells undergo clonal expansion and differentiation into effector or memory T-lymphocytes when meet the antigen, determining acquired responses which are at the base of the immunological memory and therefore of vaccinations. The total amount of naive T-lymphocytes which received the "priming" relates to the extent of immune responses generated against a determined antigen (6). Recent studies show that T lymphocytes expansion does not depend on precursor T-lymphocytes frequency, but it is linearly proportional to the total amount of progenitor T naive cells (12).

In a previous study, inventors have identified a new mechanism of immune regulation by means of which one T- lymphocytes subpopulation with regulatory function (Treg) induces the increase of immune responses after immunization with tetanus toxoid used as antigen and the cholera toxin used as mucosal adjuvant (17, 19). In this study has been observed that such immune response increase correlates to Treg capability to sequester immune system cells in immunization draining lymph nodes. The study of the mechanism of action of this new and unexpected Treg function is still unknown and under study. It is widely described that Treg cells carry out a suppressive function of immune system.

It is known that lymphocytes egress from thymus or secondary lymphoid organs is regulated by sphingosine 1-phosphate, which consists of one bioactive sphyngolipid involved in many cellular processes which include cells proliferation, survival and migration (16). Sphingosine 1-phosphate binds to 5 different receptors which activate S1 P1-S1 P5 (18) G proteins, which differs for signals to be translated and for their different distribution in different tissues.

The inventors of the present invention have found that sphingosine 1-phosphate receptor modulators, which regulate leukocytes traffic, are able to improve immune responses after mucosal and systemic vaccination, when modulators are administered during a particular time slot, namely at the time of period in which antigen presenting cells have reached and are still in draining lymph nodes. Time slot refers to period of time both after vaccination and after vaccine recalls and it corresponds to the period of time in which specific vaccine cells program themself. Advantageous effects of the administration of sphingosine 1-phosphate receptor modulators are obtained for both preventive and therapeutic vaccines. The present invention is directed to preventive vaccines. However, even though vaccine is administered in a therapeutic way, the administration of sphingosine 1-phosphate receptor modulators during the time slot after vaccine administration is able to improve immune responses, since specific antigen cells from naive cells can be generated even after exposure to pathogen. The explanation about the advantageous effect of the administration of sphingosine 1-phosphate receptor modulators on immune responses after preventive or therapeutic vaccination, can be found in modulators capability to favor the sequestration of antigen presenting cells in lymph nodes and the interaction thereof with naive lymphocytes (priming). Particularly, it has been found that the treatment with sphingosine 1-phosphate receptor modulators, as for example FTY720 compound, increases tetanus specific responses both at systemic and mucosal level, as a consequence of tetanus toxoid or ovalbumin vaccination used as antigens and cholera toxin used as adjuvant.

FTY720 is a sphingosine analogue, 2-amino-2-(2-(4-octylphenyl)ethyl)propane-1,3-diol hydrochloride) and it is a synthetic myrocin derivative, a metabolite of *Isaria sinclairii* fungus and it acts like a strong agonist for 4 of 5 S1P receptors (S1P1, S1P3, S1P4 e S1P5) (2, 13). FTY720 has been authorized as drug (Gilenya™) for the treatment of Multiple Sclerosis (1) by the American FDA, through its capability to inhibit lymphocytes egress from lymph nodes.

FTY720 induces T lymphocytes accumulation in the secondary lymphoid organs preventing in such a way active cells to reach the central nervous system and causing damages (1), through the bond to its receptors. FTY720 has been classified as new immunity repressor class, however, it does not inhibit cell effector functions. In fact, it has been demonstrated that said compound does not interfere with cells and humoral immune responses induction, expansion and generation (15). Lymphocytes sequestration through FTY720 in lymphoid organs is reversible, and that allows lymphocytes to return to circulation and carry out their function when the treatment has finished (14). Furthermore, several agonists specific for different S1P receptors, which target different tissues selectively and are currently being studied for the treatment of several diseases (16), have been identified.

WO2008/124210 discloses the use of sphingosine-1-phosphate receptor agonists including FTY720 as adjuvants in methods of immunization. FTY720 is used for post-exposure prophylaxis or for the treatment of an established chronic infection.

CIABATTINI ANNALISA ET AL: "Distribution of Primed T Cells and Antigen-Loaded Antigen Presenting Cells Following Intranasal Immunization in Mice", vol. 6, no. 4, April 2011, pages 1-8, discloses the use of CpG ODN as mucosal vaccine adjuvant in preventive vaccine immunization. According to the present invention, after vaccination, FTY720 treatment increases vaccine specific immune responses and therefore it can be used as coadjuvant for reducing doses and increasing new or already available vaccines effectiveness with a good safety profiles.

APCs recruitment and mobilization are crucial events in T lymphocytes priming, after both mucosal and systemic vaccination. Dendritic cells play a major role in controlling and beginning the immune responses. As FTY720 also influences DCs migration (10, 11), it is necessary to start drug administration after intranasal or systemic immunization, in order to allow DCs to migrate to draining lymph nodes and beginning antigen presentation to T cells. The time slot subsequent to vaccine administration may be defined in a functional way as it ranges depending on the nature of used vaccine or adjuvant. The time slot for the administration of S1P receptor modulator depends on how many time presenting antigen cells spend for leaving the peripheral tissue (which is the vaccine inoculum) and reaching the draining lymph nodes after vaccination. Such period of time may be valued in the range from 6 to 72 hours after the administration of vaccine and each recall. As far as based on living organism or slow-release vaccines are concerned, it could be hypothesized a drug administration with intervals of 1, 2, 3 or 4 weeks.

In general, data deriving from phase III clinical trials, in which two doses of FTY720 (fingolimod) by oral administration has been valued (0.5 and 1.25 mg a day) for the treatment of patients affected by Multiple Sclerosis, have shown that drug was effective and well tolerated by patients (5, 8, 9). Mainly in patients who received the higher dose, some infectious complications have been observed. However, fingolimod has been released by FDA and it is currently used for the treatment of Multiple Sclerosis, thanks to its high effectiveness with a good safety profiles. Noteworthy is the fact that, the treatment of Multiple Sclerosis patients requires the administration of fingolimod for a prolonged period, whereas the study now carried out by the inventors show that drug administration, after vaccination and in the as above mentioned time slot, increases the immune responses with effects of long duration. The administration condition of the as above mentioned drug is generally carried out orally, as it is currently performed to Multiple Sclerosis patients, and dose may be that one clinically approved (0.5 mg/dose) or that one already used for the clinical trial (1.25 mg/dose). Preferably, drug administration could be carried out during a period of time which may range from 6 to 72 hours after the administration of vaccine and each recall. However, suitable clinical test, in order to monitor the treatment effectiveness in the vaccination context, should be performed. Indeed, the use of fingolimod in the vaccination strategy requires the treatment to be interrupted for allowing to memory cells, generated at the priming moment, to go into the circulation and reach peripheral tissues.

Therefore sphingosine 1-phosphate receptor modulators can be used as immune coadjuvants in preventive or therapeutic vaccine immunizations, wherein said modulators are administered in a period of time, after the administration of vaccine and each recall, in which vaccine specific naive T lymphocytes and antigen presenting cells have reached and are still in draining lymph nodes.

Specifically, the present invention concerns a sphingosine 1-phosphate receptor modulator for use as immune coadjuvant in preventive vaccine immunizations, wherein said modulator is FTY720 and is administered in a period of time that ranges from 6 to 72 hours after the administration of vaccine and each recall.

Vaccines may be distinguished in preventive when the subject is sane, is not infected or there is not disease and therapeutic when they are administered to infected or sick people.

As immune coadjuvants are intended substances which differ from vaccine coadjuvant in the strict sense as they have distinct immunological targets and they do not replace classic adjuvants, but they enhance effectiveness thereof. An adjuvant enhances vaccinations and it may do that at different levels: ì) acting on APC permitting them to mature, migrate, polarize, present better; ìì) enhancing antigen delivery; ììì) summoning other cells and determining a proinflammatory cascade. Coadjuvant does not exclude the use of an adjuvant. In fact, coadjuvant may enhance adjuvant effect. In the case of the present invention, the coadjuvant favors the priming of vaccine-specific cells forcing them to stay physically in the same place and receive necessary stimuli.

Sphingosine 1-phosphate receptor modulators are known to the skilled in the art, like so tests for verifying that one compound is a S1P modulator (15, 21, 22). In fact among S1P receptor modulators are comprised: agonists which bind to S1PR receptors, allowing their internalization and preventing Sphingosine 1-phosphate to bind (like FTY720, KRP203, AUY954, CYM-5442 SEW2871); antagonists which bind to receptors and prevent Sphingosine 1-phosphate to bind; sphingosine kinase inhibitors, the enzyme that regulates Sphingosine 1-phosphate (much used in tumors field) and also one humanized S1P monoclonal antibody (LT1009, for cancer treatment at Phase I), which prevents S1P bond with receptors. More in detail, sphingosine 1-phosphate receptor modulators are the following receptor agonists: FTY720 (2-amino-2[2-(4-octylphenyl) ethyl] propane-1,3-diol), AUY954 (S1P1 selective) (1-hydroxy-4-[({2-[4-phenyl-3-(trifluoromethyl)phenyl]-1-benzothiophen-5-yl}methyl)amino]butan-2-one), CYM-5442 (S1P1 selective) (2-({4-[5-(3,4-dietossiphenyl) -1,2,4-oxadiazol-3-yl] -2,3-dihydro-1H-inden-1-yl} amino) ethane-1 -ol), KRP-203 (S1P1 selective) (2-amino-2-(4-((3-(benzyloxy)phenyl)thio)-2-clorofenetil)propane-1,3-diol hydrochloride), SEW2871 (S1P1 selective) (5-[4-phenyl-5-(trifluoromethyl)thiophen-2-yl]-3-[3-(trifluoromethyl) phenyl]1,2,4-oxadiazole), and the following receptor antagonists: JTE 013 (specific for S1 P2) (3-(2,6-dichloropyridine-4-yl)-1-[(1,3-dimethyl-4-propan-2-yl pyrazole[4,5-e]pyridin-6-yl)amino]urea), VPC 23019 (specific for S1P1 and S1P3) ([(2R)-2-amino-2-[(3-octilphenyl)carbamoyl]ethoxy] phosphonic acid).

Particularly, FTY720 may be administered in dose from 0.5 to 1.25 mg/dose, whereas KRP203 dose is 1.2 mg. Modulators way of administration, according to the present invention, is preferably oral.

Even more preferably modulators may be administered from 1 to 3 times after the administration of vaccine and each recall, each time in a single daily dose for only once a day. For example, the modulator may be administered only once the day after vaccine administration and/or only once two days after and/or only once three days after. Vaccine may be for both human and veterinary use. The vaccine may be selected from the group consisting of vaccine against diphtheria, viral hepatitis, rubella, meningitis, HPV human papilloma virus, tetanus, poliomyelitis, measles, whooping cough, mumps, varicella, tuberculosis, seasonal and pandemic influenza, international travelers diseases among which typhoid fever, cholera, amebiasis, malaria, yellow fever, Dengue, Japanese encephalitis, Hantavirus infections, Rift Valley Fever, Sars, severe acute respiratory syndrome, West Nile virus infection, Chikungunya, Marburg hemorrhagic fever.

Constitute a further object of the present invention a combination of sphingosine 1-phosphate receptor modulator FTY720 with at least one vaccine for the sequential use as immunological coadjuvant in preventive vaccine immunizations, wherein said modulator is administered in a period of time that ranges from 6 to 72 hours after the administration of the vaccine and each recall, wherein vaccine specific naive T lymphocytes and antigen presenting cells have reached and are still in draining lymph nodes.

The vaccine may be for both human and veterinary use. Among vaccines, the following may be mentioned: vaccine against diphtheria, viral hepatitis, rubella, meningitis, HPV human papilloma virus, tetanus, poliomyelitis,
measles, whooping cough, mumps, varicella, tuberculosis, seasonal and pandemic influenza, international travelers diseases among which typhoid fever, cholera, amebiasis, malaria, yellow fever, Dengue, Japanese encephalitis, Hantavirus infections, Rift Valley Fever, Sars, severe acute respiratory syndrome, West Nile virus infection, Chikungunya, Marburg hemorrhagic fever. As mentioned above, the time slot in which the according to the invention modulator is administered, ranges from 6 to 72 hours after the administration of vaccine and each recall. According to a particular embodiment of the present invention, said modulator is administered from 1 to 3 times after the vaccine administration and each recall, each time in a single daily dose for only once a day. For example, the modulator may be administered only once the day after vaccine administration and/or only once two days after and/or only once three days after.

FTY720 may be administered in dose from 0.5 to 1.25 mg/dose, whereas KRP203 dose is 1.2 mg. The preferable way of administration is the oral way.

The present invention now will be described by an illustrative way according to preferred embodiments thereof, with particular reference to enclosed drawings.

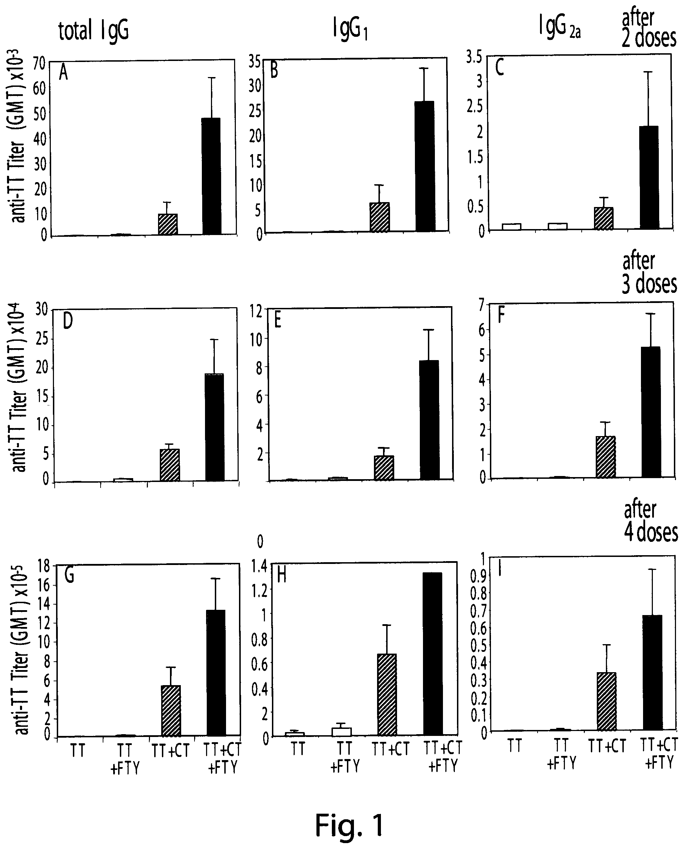

**Figure 1****.** Antibody response at systemic level after TT and CT intranasal immunization followed by FTY720 treatment. IgG, IgG1 and IgG2a levels specific for TT in serum of BALB/c mice, intranasally immunized with TT, TT followed by FTY720, TT+CT or TT+CT followed by FTY720 treatment. The analysis was performed one week after the second, the third and the fourth immunization. The results are expressed as the geometric mean of titres of specific IgG (GMT) of 6-8 mice for group and bars indicate the standard errors between mice of the same group.

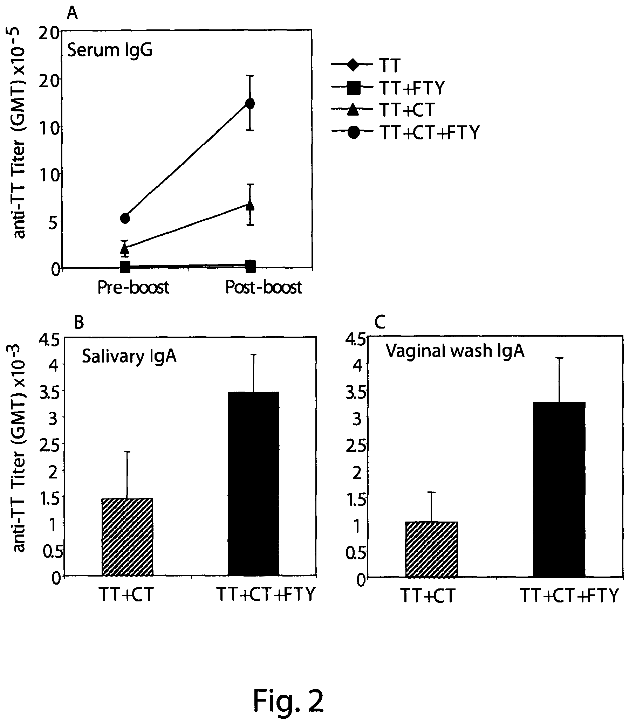

**Figure 2****.** Antibody response at systemic and mucosal level after intranasal immunization with TT and CT followed by FTY720 treatment. Serum IgG levels and IgA in mucous specific for TT of BALB/c mice intranasally immunized with TT+CT or TT+CT followed by FTY720 treatment. The analysis was performed four months after the fourth immunization (pre-boost) and one week after the recall dose (post-boost) for in serum IgG (A) and one week after the recall dose for the mucosal IgA (B and C). The results are expressed as the geometric mean of the titres of specific IgG and IgA (GMT) of 6-8 mice for group and bars indicate the standard errors between mice of the same group.

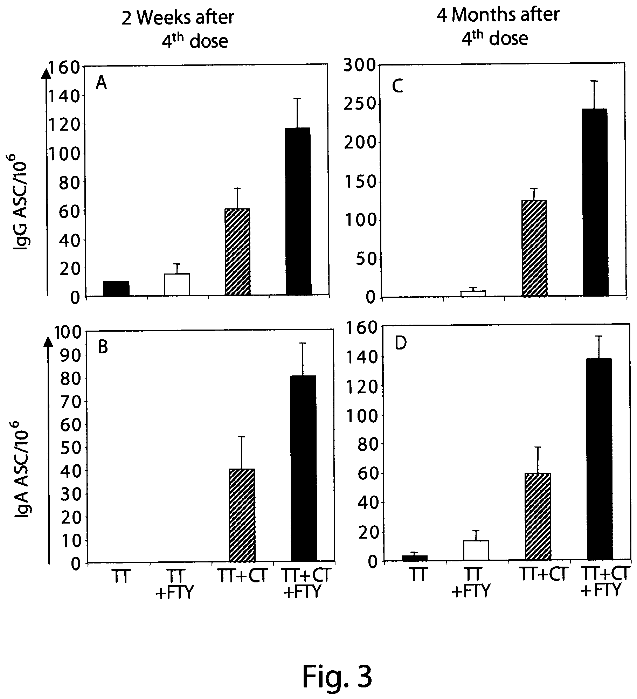

**Figure 3****.** Analysis of antibody secreting cells (ASC) specific for the TT in the bone marrow. The presence of ASC IgG (above panels) and ASC IgA (below panels) specific for the TT was valued in the bone marrow cells (pool of cells from 4 mice of the same group) by ELISPOT 2 weeks after the fourth immunization (panels on the left), and 2 weeks after the recall dose, administered 4 months after the fourth administration (panels on the right) with TT, TT followed by treatment with FTY720, TT+CT or TT+CT followed by FTY720 treatment. The results of a representative experiment are expressed as number of ASC specific for TT for 1x10⁶ bone marrow derived cells. Bars indicate the standard deviations of duplicates of the same experiment.

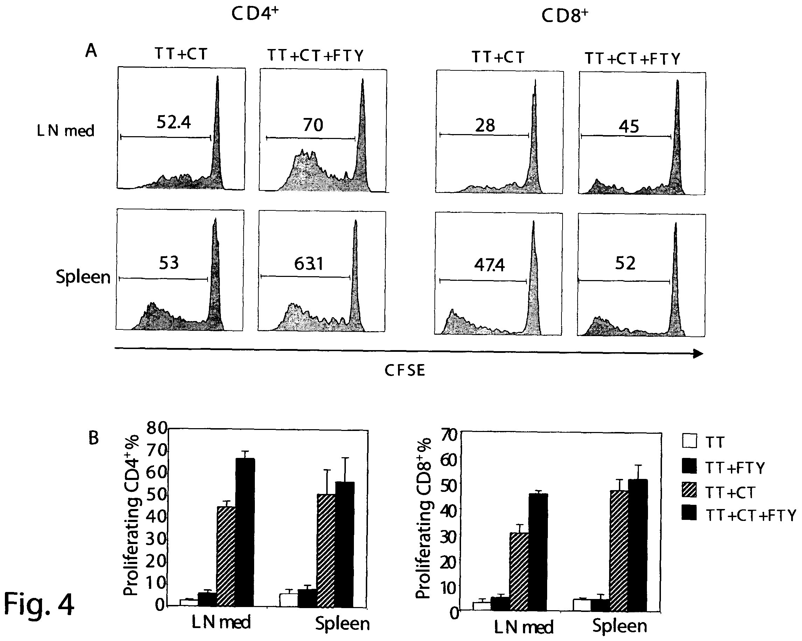

**Figure 4****.** Analysis of the proliferation of antigen-specific CD4⁺ and CD8⁺ T lymphocytes in different districts. BALB/c mice were intranasally immunized with TT+CT or TT+CT followed by FTY720 treatment. Cells deriving from mediastinal lymph nodes and spleens were harvested 2 weeks after the fourth immunization, marked with CFSE, and cultivated in vitro for 5 days in the presence and absence of TT (2 µg/ml), marked with anti-CD4-PE or anti-CD8-PercP monoclonal antibody and analyzed by flow citometer. (A) It is showed a FACS analysis of a representative experiment. The percentage of proliferating TT-specific cells was calculated by CFSE dilution in CD4⁺ (panels on the left) and CD8⁺ (panels on the right) population. (B) The mean of proliferating cells percentages within CD4⁺ (panels on the left) and CD8⁺ (panels on the right) population is reported. Bars indicate the standard errors between mice of the same group.

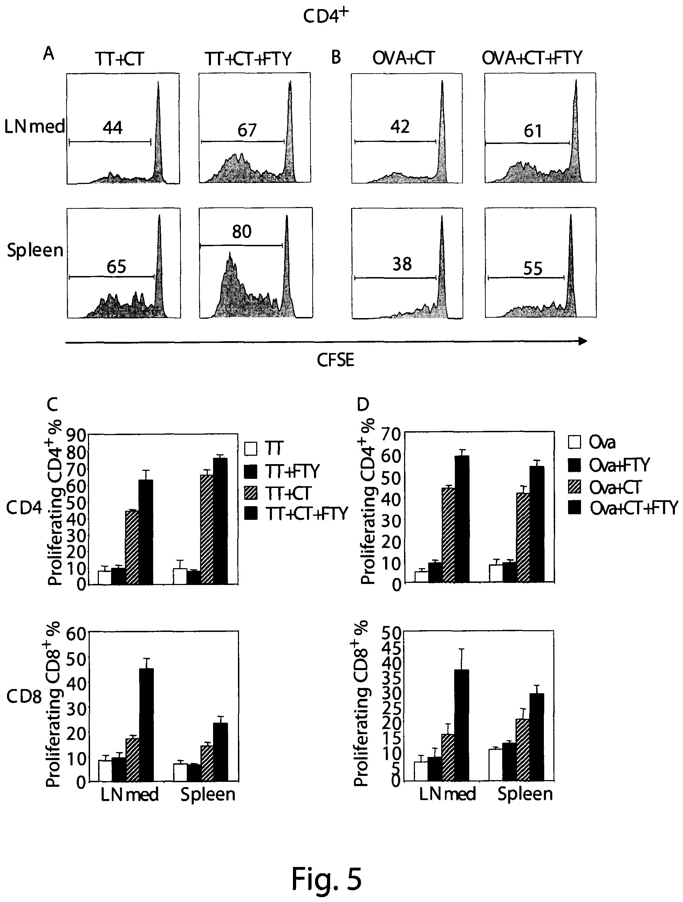

**Figure 5****.** Analysis of the proliferation of antigen-specific CD4⁺ and CD8⁺ T lymphocytes in different districts. BALB/c mice were intranasally immunized with TT (A, C) or Ova (B, D) as antigens and CT as adjuvant in the presence and absence of FTY720. Cells deriving from mediastinal lymph nodes and spleens were harvested 8 months after the fourth immunization after one recall dose, marked with CFSE, cultivated in vitro for 5 days in the presence and absence of TT (2 µg/ml) or Ova (2 µg/ml), marked with monoclonal antibody anti-CD4-PE or anti-CD8-PercP and analyzed by flow cytometer. It is showed a FACS analysis of a representative experiment. The percentage of TT specific (A) and Ova-specific (B) proliferating cells was calculated by the dilution of CFSE in CD4⁺ population. The mean of proliferating TT-specific (C) and Ova-specific (D) cell percentages, calculated within CD4⁺ (above panels) and CD8⁺ (below panels) population, is reported. Bars indicate the standard errors between mice of the same group.

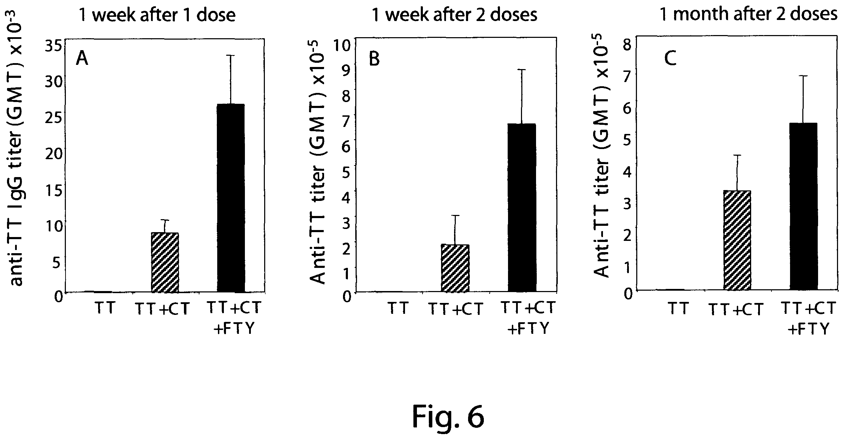

**Figure 6****.** Antibody response at systemic level after systemic immunization with TT and CT followed by FTY720 treatment. IgG specific levels for TT in serum of BALB/c mice immunized intraperitoneally with TT, TT+CT or TT+CT followed by FTY720. The analysis was performed one week after the first dose (A), one week (B) and one month (C) after the second dose. The Results are expressed as the geometric mean of specific IgG titres (GMT) of 4-6 mice for group and bars indicate the standard errors between mice of the same group.

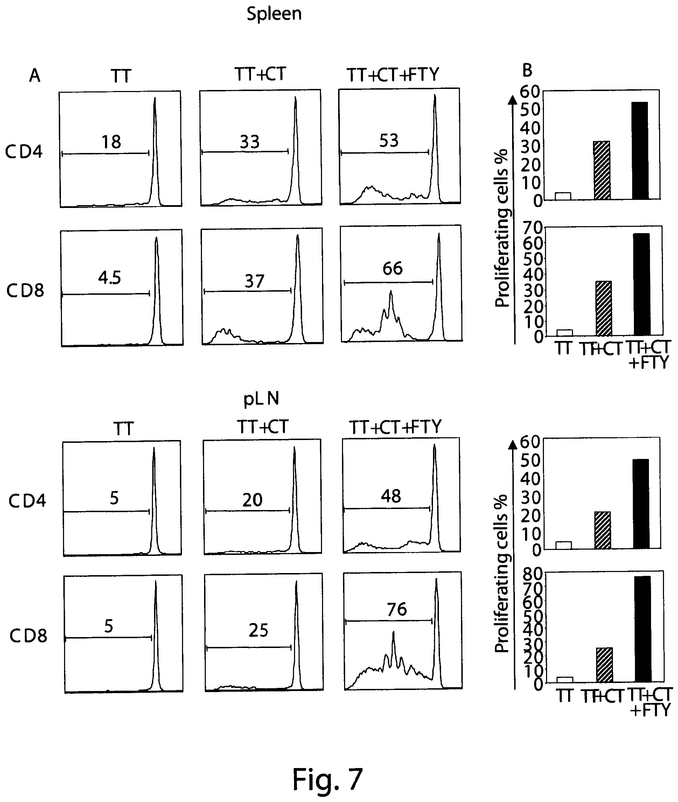

**Figure 7****.** Analysis of the proliferation of antigen-specific T CD4⁺ e CD8⁺ T-lymphocytes in different districts. BALB/c mice were immunized intraperitoneally with TT as antigen and CT as adjuvant in the presence and absence of FTY720. Cells deriving from mediastinal lymph nodes and spleens were harvested 4 months after the second immunization, marked with CFSE, cultivated in vitro for 5 days in the presence and absence of TT (2 µg/ml), marked with anti-CD4-PE or anti-CD8-PercP monoclonal antibody and analyzed by flow cytometer. One FACS analysis of a representative experiment (A) is showed. The percentage of proliferating TT-specific cells was calculated by the dilution of CFSE in CD4⁺ and CD8⁺ population. The mean of proliferating TT-specific cell percentages (B), calculated within CD4⁺ and CD8⁺ population, is reported. Bars indicate the standard errors between mice of the same group.

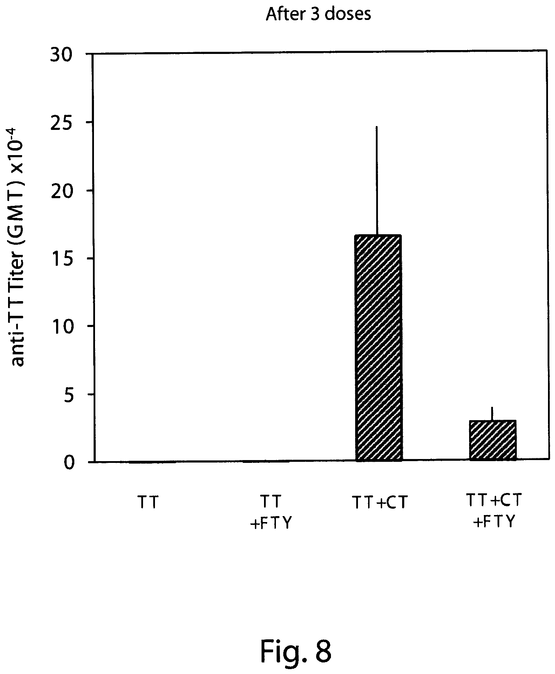

**Figure 8****.** Antibody response at systemic level after treatment with FTY720 drug, administered 48 and 24 hours before the intranasal immunization with TT and CT. IgG levels, specific for TT in serum of BALB/c mice intranasally immunized with TT, TT preceded by treatment with FTY720, TT+CT or TT+CT preceded by FTY720 treatment. The analysis was performed one week after the third immunization. The results are expressed as the geometric mean of specific IgG titres (GMT) of 4-5 mice for group and bars indicate the standard errors between mice of the same group.

**EXAMPLE 1:** Study in vivo on FTY720 effects in vaccine immunizations.

### Materials and methods

*Medium and reagents.* RPMI 1640 supplemented with 2 mM L-glutamine, 1% (v/v) non-essential amino acids, 1% (v/v) pyruvate, 55 µM β2-mercaptoethanol 100 U/ml penicillin, 100 µg/ml streptomycin (Gibco, USA), e 10% FCS (Hyclone Laboratories, USA), was used as complete medium in all the cultures. Anti-CD3 monoclonal antibody (clone 145-2C11) (mAb) has been purchased by BD Biosciences, (USA). Tetanus toxoid (TT) was made by Novartis (Italy), CT and FTY720 were purchased by Calbiochem-Novabiochem (USA), ovalbumin (Ova) was purchased by Sigma Aldrich, USA.

*Intranasal and systemic immunizations.* Female BALB/c mice aged 6-8 weeks were obtained by harlan Nossan, Italy and maintained in our enclosures for all the duration of the experiments. All the procedures relating to the treatment of animals are in accordance with the institutional guidelines. Groups of 4 mice were intranasally immunized for four times at weekly intervals (day 0, 7, 14 and 21), with 1 µg of tetanus toxoid (TT) or ovalbumin (Ova) in the presence or absence of CT (0.5 µg/dose). As far as the immunization by systemic way is concerned, TT (1 µg/dose) were administered intraperitoneally in the presence or absence of CT (0.5 µg/dose) two times at weekly intervals (days 0 e 7). The day after the first and the second immunization both mucosal and systemic, some groups of mice were treated with FTY720 (0.5 mg/Kg) or vehicle thereof by intragastric administration. In some experiments, mice received one recall dose of antigen and adjuvant, 4 or 8 months after the fourth immunization. As far as the intranasal immunization is concerned, mice were lightly anesthetized by means of one Ketamine (Ketavet, Gellini Pharmaceutics, 50 mg/Kg of weight) and xylazine (Rompun, Bayer, 3 mg/Kg of weight) intraperitoneal injection. A volume of 15-20 µl (i.e., 7.5-10 µl/nostril) of a solution of PBS containing the antigen with or without the adjuvant was administered.

*Samples harvesting.* Serum samples were obtained by peripheral blood harvested by the retro-orbital plexus of anesthetized mice and then frozen and kept at -20°C. Salivae were harvested after one peritoneal injection of pilocarpine (100 µg/animal). Vaginal washes were obtained introducing 50 µl of PBS for three times into the vaginal tract, using a Gilson pipette. Serum was harvested one week after the second, third and fourth immunization, 4 months after the fourth immunization and one week after the recall dose. Salivae and vaginal washes were harvested 4 months after the fourth immunization, one week after the recall dose. After systemic immunization sera, were harvested one week after the first immunization and one week and one month after the second immunization.

*Analysis of antibody isotopes.* All the samples were tested by Elisa test. 96 well plates, (Greiner bio-one, Germany) were incubated with 0.5 µg/well of TT for one night at 4° C. After one wash with PBS 0.05% (v/v) Tween 20 (UCS Diagnostics, Italia) and 2 h rest with 200 µl of PBS containing 1% (v/v) of bovine serum albumin (Sigma Aldrich, USA), serial dilutions of single mice sera or mucosal fluids were added in duplicate and incubated for 2 h at room temperature. The plates were washed and anti-IgG or anti-IgA antibodies of biotinylated mouse (Sigma Aldrich, USA) diluted in PBS-Tween 20 were added in wells and incubated for 2h at room temperature. The plates were washed and streptavidin conjugated with HRP (Dako, Italy) diluted 1:2000 in PBS-Tween for 30 minutes at room temperature were added. Finally, after washes with PBS-Tween, antigen antibody reaction was measured using 3.3, 5.5 - tetramethylbenzidine (Kirkegaard & Perry Laboratories, USA) as substrate. The coloring of the reaction was arrested after 5-10 minutes with 50 µl of 0.2 M H₂SO₄. The final titres were determined as the reciprocal of the higher dilution which corresponds to an absorbance of more than 0.3 units (as regards IgG) and 0.2 units (as regards IgA) with respect to the negative controls.

*ELISPOT antibody secreting cells (ASC).* Cells were isolated by the bone marrow by RPMI flow from one of the ends of the femur or tibia. Bone marrow cells derived from mice of the same group were merged and tested at the frequency of TT-specific ASC by ELISPOT assay. 2×10⁵ cells/well were incubated for one night at 37°C in 96 well plates (PVDF) (Millipore), incubated with TT (20µg/ml) from the day before. Then plates were abundantly washed and developed with goat anti-mouse IgG and IgA antibody HRP conjugated (Sigma Chemicals, Co., St. Louis, MO, USA) followed by incubation with a chromogenic substrate (Aminoethylcarbazole) (Sigma Chemicals, Co., St. Louis, MO, USA). Spots were counted by automatic reader (A.EL.VIS, Hannover, Germany).

*Proliferation assay.* Splenocytes and lymph node derived cells were obtained by mechanical failure of organs and filtered by membranes with pore diameter of 70-100 µm (BD Pharmingen, San Diego, CA, USA). Cells were washed with PBS 1% FBS and incubated with 2.5µM di carboxyfluorescein succinimidyl ester (CFSE, Molecular probes, Eugene, USA) for 10 min at 37 °C. The reaction were interrupted by adding cold complete medium and incubated for 5 min at 4°C. Cells were washed 2 times by cold complete medium and plating with TT or Ova (2µg/ml), anti-CD3 monoclonal antibody (mAb) (0.1 µg/ml), (BD Pharmingen, San Diego, CA, USA) or without stimuli at a concentration of 2×10⁵ cells/well in 96-well plates (Costar, USA). After 5-6 days, the cells were marked with anti-CD4-PE or anti-CD8-PercP monoclonal antibody (BD Pharmingen, San Diego, CA, USA) and acquired by flow cytometer FACSCaliburTM and analyzed by dedicated software (CellQuest or flowjo).

*Statistical analysis.* Data were expressed as geometric mean or mean ± standard error and significance has been calculated by Student's t test. When p calculated comparing two groups of values was < 0.05, differences was considered significant.

### Results

### FTY720 treatment following the vaccination with tetanus toxoid and cholera toxin increases tetanus-specific antibody response.

In the attempt to identify a new approach for improving vaccine immunizations, we have investigate a new class of immune agonists which have the capability to hold immune system cells in lymph nodes. The purpose of our job is to improve immunization directing cells of the immune system to draining lymph nodes, where the "priming " takes place and therefore the generation of immune responses against specific antigens. Balb/c mice were intranasally immunized with tetanus toxoide (TT) (1 µg/dose) and with or without cholera toxin used as mucosal adjuvant (CT) (0.5 µg/dose) for four times, once a week. The day after the first and the second immunization, some groups received FTY720 (0.5 mg/Kg) or the vehicle by means of intragastric administration. TT-specific antibody responses were analyzed one week after the second, the third and the fourth immunization. After the second immunization, the anti-tetanus IgG antibody titre in mice immunized with TT and CT and treated with FTY720 was higher (305 times with respect to the titre of mice which received only TT) when compared to the titre of mice immunized with TT and CT in the absence of treatment (54 times with respect to the titre of mice which received only TT) (Figure 1A). Such an increase, observed in mice which received the drug, was observed even after the third (Figure 1D) and the fourth (761 vs 304) (Figure 1G) immunization. Furthermore, the immunoglobulin subclasses (lgG1 and IgG2a) were analyzed after immunization with TT and CT in the presence and absence of FTY720 treatment. An Increase of the titre of both IgG1 and IgG2a subclasses was observed in mice immunized with TT and CT and treated with FTY720 at different analyzed kinetic points (Figure 1).

### Persistence of antigen-specific immune responses after FTY720 treatment following the vaccination.

The anti-tetanus IgG antibody titre, in the attempt to assess the persistence of antigen-specific antibody responses in mice immunized with TT and CT in the absence and the presence of FTY720 treatment, four months after the fourth immunization, was valued. The anti-TT IgG antibody titre was decreased when compared to that one observed after the fourth immunization, however specific antibodies were still detectable and maintained at higher levels in mice immunized and treated with FTY720 (Figure 2A, pre-boost). Later, memory was valued and the different groups received a boost four months after the fourth immunization and both systemic and mucosal antibody responses were analyzed. Once again, mice which received the FTY720 treatment after immunization showed higher levels of IgG antibodies specific for the TT when compared to immunized mice which have not received the treatment (Figure 2A).

In the attempt to value the response at mucosal level, the titre of TT-specific IgA antibodies was measured in fluids of two mucosal districts, saliva and vaginal washes (VW). As showed in figure 2BC, antigen-specific IgA antibodies were found in saliva and VW and higher titres were measured in mice immunized with TT and CT and treated with FTY720 after immunization.

### Analysis of antigen-specific antibody-secreting B cells after immunization and FTY720 treatment.

In order to analyze the effect of FTY720 treatment on cellular immune response in mice immunized with TT and CT, the presence of TT-specific antibody secreting cells (ASC) in cellular suspensions derived from the bone marrow (BM), two weeks after the fourth immunization and two weeks after the recall dose occurred 4 months after the fourth immunization (boost), was valued by means of ELISPOT. As showed in figure 3A, an higher number of anti-TT IgG ASC was observed in the bone marrow of immunized and FTY720 treated mice with respect to the bone marrow of immunized and not treated mice (115 and 60 ASC/1×10⁶ of cells, respectively), two weeks after the fourth immunization. The number of specific ASCs was found increased after the recall dose in both groups and significant differences were observed between groups treated with FTY720 and non treated groups (Figure 3C) (240 and 123 ASC/1×10⁶ of cells, respectively). Anti-TT IgA secreting cells were detected in the bone marrow of mice immunized at both the analyzed times (Figure 3B and D). Particularly, mice treated with FTY720 show an higher number of IgA ASC with respect to immunized and non treated animals, respectively 80 and 40 ASC/1×10⁶ of cells, two weeks after the fourth immunization (Figure 3B) and respectively 136 and 58 ASC/1×10⁶ of cells, two weeks after the recall dose (Figure 3D). Both IgG and IgA ASC of treated and non treated groups are statistically different from the groups which have received TT or TT plus FTY720 in the absence of CT.

### Analysis of tetanus-specific T lymphocyte responses.

In order to assess the presence of antigen-specific T lymphocytes, mediastinal lymph nodes, which were described as being lymph nodes draining the intranasal immunizations (20), and spleens were processed. Cells were harvested 2 weeks and 8 months after the fourth immunization and were used in a proliferation test. Both CD4⁺ and CD8⁺ populations specific for the TT were analyzed. Cells isolated from spleen and lymph nodes were marked with CFSE and cultivated in the presence of TT for 5 days. The percentage of proliferating cells was calculated within CD4⁺ or CD8⁺ populations. Cells stimulated with anti-CD3 monoclonal antibodies, were included as positive control and proliferated as it was expected (between 60% and 90%, data not showed). The mice, intranasally immunized with TT and CT and treated with FTY720, show a proliferation of antigen-specific CD4⁺ and CD8⁺ T lymphocytes significantly higher with respect to immunized and non treated mice (p < 0.05) (Figure 4) two weeks after the fourth immunization in draining lymph nodes. The proliferation of antigen-specific CD4⁺ and CD8⁺ T lymphocytes in the spleen, two weeks after the fourth immunization, was comparable between cells isolated from mice immunized with TT and CT treated and non treated with FTY720 (Figure 4).

In order to test the durability of the immune cellular responses induced by vaccination, T lymphocyte proliferation was valued 8 months after the fourth immunization, one week after one recall dose (Figure 5). As observed before, the proliferation of antigen-specific CD4⁺ and CD8⁺ T lymphocytes in draining lymph nodes of mice immunized with TT and CT and treated with FTY720, was higher with respect to mice immunized and non treated (p < 0.05) (Figure 5). Unlike what previously observed, at this kinetic point, an higher percentage of antigen-specific CD4⁺ and CD8⁺ T lymphocytes was observed even in cells deriving from spleen of mice immunized and treated with FTY720 with respect to non treated mice. In order to assess the capability of FTY720 to modulate the immune responses against a different antigen, mice were immunized with ovalbumin (Ova) following the same as above described immunization strategy and the presence of Ova-specific CD4⁺ CD8⁺ T lymphocytes was analyzed 8 months after the fourth immunization. In a similar way to TT-specific T responses, an higher percentage of Ova-specific CD4⁺ and CD8⁺ T lymphocytes in cells deriving from both draining lymph nodes and spleen of mice immunized and treated with FTY720, with respect to non treated mice, was observed, suggesting that the modulation of immune responses by FTY720 is not TT-restricted.

### FTY720 treatment following one systemic vaccination with tetanus toxoid and cholera toxin increases antibody and cellular specific responses.

We performed experiments using TT as antigen and CT as systemic adjuvant, in order to assess whether the effects of FTY720 treatment in improving responses induced by vaccination were linked to the mucosal immunization way or whether it could be applied to a systemic immunization. Balb/c mice were immunized intraperitoneally with TT (1 µg/dose) and with or without CT (0.5 µg/dose), which is known for being even an excellent systemic adjuvant, two times at one week of distance.

The day after the first and the second immunization, some groups of mice received FTY720 (0.5 mg/Kg) or vehicle thereof intragastrically. Antibody responses in serum were examined one week after the first immunization and one week and one month after the second immunization. After the first immunization, titre of anti-tetanus IgG antibodies in sera of mice immunized with TT plus CT and treated with FTY720 was higher (203 times with respect to the titre of mice immunized only with TT) with respect to the titre of mice immunized with TT and CT in the absence of treatment (63 times higher) (Figure 6A). An higher increase, in mice which received the drug, was maintained one week after the second immunization (575 vs 152) (Figure 6B). Differences between the two groups decreased, but they were still detectable (Figure 6C), one month after the second immunization.

Subsequently, immunization draining lymph nodes and spleen were harvested 4 months after the second immunization, in order to analyze the presence of antigen-specific T lymphocytes. Both T CD4⁺ e CD8⁺ populations were analyzed. Cells were marked with CFSE and cultivated with TT (2 µg/ml) for 5 days. The percentages of proliferating cells were calculated within CD4⁺ e CD8⁺ populations. Mice immunized with TT and CT and treated with FTY720 showed an higher percentage of antigen-specific CD4⁺ and CD8⁺ proliferating cells in lymph nodes and spleen with respect to immunized and non treated mice (p < 0.05) (Figure 7).

### The FTY720 treatment before vaccination with tetanus toxoid and cholera toxin inhibits the tetanus-specific antibody responses.

In another experiment set we valued the effect of the treatment with sphingosine analogues administered before the vaccine administration. Balb/c mice were treated with FTY720 (0.5 mg/Kg) or vehicle thereof by intragastric administration, 48 and 24 hours before receiving the intranasal immunization with tetanus toxoid (TT) (1 µg/dose) and with or without cholera toxin used as mucosal adjuvant (CT) (0.5 µg/dose). Mice received the vaccine for other two times, once a week. TT-specific antibody responses were analyzed one week after the second and the third immunization. After the second immunization only the titre of anti-tetanus IgG antibodies in mice immunized with TT and CT was appreciable with respect to the other groups (data not showed). After the third immunization, the titre of IgG antibody specific for the TT was higher in mice immunized with TT and CT in the absence of treatment when compared to others groups (Figure 8). This data show that the treatment with analogues of sphingosine 1-phosphate has to be carried out after the vaccination and that may be explained with the fact that waiting for the functional time is necessary in order antigen presenting cells to reach draining lymph nodes.

### References

1. Brinkmann, V., A. Billich, T. Baumruker, P. Heining, R. Schmouder, G. Francis, S. Aradhye, and P. Burtin. Fingolimod (FTY720): discovery and development of an oral drug to treat multiple sclerosis. Nat Rev Drug Discov 9:883-897.
2. Brinkmann, V., M. D. Davis, C. E. Heise, R. Albert, S. Cottens, R. Hof, C. Bruns, E. Prieschl, T. Baumruker, P. Hiestand, C. A. Foster, M. Zollinger, and K. R. Lynch. 2002. The immune modulator FTY720 targets sphingosine 1-phosphate receptors. J Biol Chem 277:21453-21457.
3. Cahalan, M. D., and I. Parker. 2006. Imaging the choreography of lymphocyte trafficking and the immune response. Curr Opin Immunol 18:476-482.
4. Catron, D. M., A. A. Itano, K. A. Pape, D. L. Mueller, and M. K. Jenkins. 2004. Visualizing the first 50 hr of the primary immune response to a soluble antigen. Immunity 21:341-34
**5.** Cohen, J. A., F. Barkhof, G. Comi, H. P. Hartung, B. O. Khatri, X. Montalban, J. Pelletier, R. Capra, P. Gallo, G. Izquierdo, K. Tiel-Wilck, A. de Vera, J. Jin, T. Stites, S. Wu, S. Aradhye, and L. Kappos. Oral fingolimod or intramuscular interferon for relapsing multiple sclerosis. The New England journal of medicine 362:402-415.
6. Galli, G., D. Medini, E. Borgogni, L. Zedda, M. Bardelli, C. Malzone, S. Nuti, S. Tavarini, C. Sammicheli, A. K. Hilbert, V. Brauer, A. Banzhoff, R. Rappuoli, G. Del Giudice, and F. Castellino. 2009. Adjuvanted H5N1 vaccine induces early CD4+ T cell response that predicts long-term persistence of protective antibody levels. Proc Natl Acad Sci U S A 106:3877-3882.
7. Henrickson, S. E., T. R. Mempel, I. B. Mazo, B. Liu, M. N. Artyomov, H. Zheng, A. Peixoto, M. Flynn, B. Senman, T. Junt, H. C. Wong, A. K. Chakraborty, and U. H. von Andrian. 2008. In vivo imaging of T cell priming. Sci Signal 1:pt2.
8. Kappos, L., E. W. Radue, P. O'Connor, C. Polman, R. Hohlfeld, P. Calabresi, K. Selmaj, C. Agoropoulou, M. Leyk, L. Zhang-Auberson, and P. Burtin. A placebo-controlled trial of oral fingolimod in relapsing multiple sclerosis. The New England journal of medicine 362:387-401.
9. Khatri, B., F. Barkhof, G. Comi, H. P. Hartung, L. Kappos, X. Montalban, J. Pelletier, T. Stites, S. Wu, F. Holdbrook, L. Zhang-Auberson, G. Francis, and J. A. Cohen. Comparison of fingolimod with interferon beta-1a in relapsing-remitting multiple sclerosis: a randomised extension of the TRANSFORMS study. Lancet neurology 10:520-529.
10. Lamana, A., P. Martin, H. de la Fuente, L. Martinez-Munoz, A. Cruz-Adalia, M. Ramirez-Huesca, C. Escribano, K. Gollmer, M. Mellado, J. V. Stein, J. L. Rodriguez-Fernandez, F. Sanchez-Madrid, and G. M. Del Hoyo. CD69 Modulates Sphingosine-1-Phosphate-Induced Migration of Skin Dendritic Cells. The Journal of investigative dermatology 131:1503-1512.
11. Lan, Y. Y., A. De Creus, B. L. Colvin, M. Abe, V. Brinkmann, P. T. Coates, and A. W. Thomson. 2005. The sphingosine-1-phosphate receptor agonist FTY720 modulates dendritic cell trafficking in vivo. Am J Transplant 5:2649-2659.
12. Linderman, J. J., T. Riggs, M. Pande, M. Miller, S. Marino, and D. E. Kirschner. Characterizing the dynamics of CD4+ T cell priming within a lymph node. J Immunol 184:2873-2885.
13. Mandala, S., R. Hajdu, J. Bergstrom, E. Quackenbush, J. Xie, J. Milligan, R. Thornton, G. J. Shei, D. Card, C. Keohane, M. Rosenbach, J. Hale, C. L. Lynch, K. Rupprecht, W. Parsons, and H. Rosen. 2002. Alteration of lymphocyte trafficking by sphingosine-1-phosphate receptor agonists. Science 296:346-349.
14. Morris, M. A., D. R. Gibb, F. Picard, V. Brinkmann, M. Straume, and K. Ley. 2005. Transient T cell accumulation in lymph nodes and sustained lymphopenia in mice treated with FTY720. European journal of immunology 35:3570-3580.
15. Pinschewer, D. D., A. F. Ochsenbein, B. Odermatt, V. Brinkmann, H. Hengartner, and R. M. Zinkernagel. 2000. FTY720 immunosuppression impairs effector T cell peripheral homing without affecting induction, expansion, and memory. J Immunol 164:5761-5770.
16. Pyne, S., and N. J. Pyne. Translational aspects of sphingosine 1-phosphate biology. Trends in molecular medicine.
17. Raghavan, S., and A. M. Harandi. 'Help' from an unexpected source: polyclonal Tregs enhance antibody response to mucosal immunization. Immunol Cell Biol 88:696-697.
18. Rosen, H., P. Gonzalez-Cabrera, D. Marsolais, S. Cahalan, A. S. Don, and M. G. Sanna. 2008. Modulating tone: the overture of S1P receptor immunotherapeutics. Immunol Rev 223:221-235.
19. Vendetti, S., T. S. Davidson, F. Veglia, A. Riccomi, D. R. Negri, R. Lindstedt, P. Pasquali, E. M. Shevach, and M. T. De Magistris. Polyclonal Treg cells enhance the activity of a mucosal adjuvant. Immunol Cell Biol 88:698-706.
20. Vendetti, S., A. Riccomi, D. R. Negri, F. Veglia, E. Sciaraffia, and M. T. De Magistris. 2009. Development of antigen-specific T cells in mediastinal lymph nodes after intranasal immunization. Methods 49:334-339.
21. von Andrian, U. H., and T. R. Mempel. 2003. Homing and cellular traffic in lymph nodes. Nat Rev Immunol 3:867-878.
22. Zielinski, C. E., D. Corti, F. Mele, D. Pinto, A. Lanzavecchia, and F. Sallusto. Dissecting the human immunologic memory for pathogens. Immunol Rev 240:40-51.

## Claims

1. Sphingosine 1-phosphate receptor modulator for use as immune coadjuvant in preventive vaccine immunizations, wherein said modulator is FTY720 and is administered in a period of time that ranges from 6 to 72 hours after the administration of vaccine and each recall.

2. Sphingosine 1-phosphate receptor modulator according to claim 1 for use according to claim 1, wherein said modulator is administered from 1 to 3 times after vaccine administration and each recall, each time in a single daily dose for only once a day.

3. Combination of sphingosine 1-phosphate receptor modulator FTY720 with at least one vaccine for the sequential use as immunological coadjuvant in preventive vaccine immunizations, wherein said modulator is administered in a period of time that ranges from 6 to 72 hours after the administration of vaccine and each recall.

4. Combination for use according to claim 3, wherein said at least one vaccine is for human or veterinary use.

5. Combination for use according to any one of claims 3-4, wherein said at least one vaccine is selected from the group consisting of vaccine against diphtheria, viral hepatitis, rubella, meningitis, HPV human papilloma virus, tetanus, poliomyelitis, measles, whooping cough, mumps, varicella, tuberculosis, seasonal and pandemic influenza, international travelers diseases among which typhoid fever, cholera, amebiasis, malaria, yellow fever, Dengue, Japanese encephalitis, Hantavirus infections, Rift Valley Fever, Sars, severe acute respiratory syndrome, West Nile virus infection, Chikungunya, Marburg hemorrhagic fever.

6. Combination for use according to any one of claims 3-5, wherein said modulator is administered from 1 to 3 times after vaccine administration and each recall, each time in a single daily dose for only once a day.

## Patentansprüche

1. Sphingosin-1-Phosphat-Rezeptor-Modulator zur Verwendung als Immun-Coadjuvans bei präventiven Impfungen, wobei der Modulator FTY720 ist und in einem Zeitraum von 6 bis 72 Stunden nach der Verabreichung des Impfstoffs und jedem Recall verabreicht wird.

2. Sphingosin-1-Phosphat-Rezeptor-Modulator nach Anspruch 1 zur Verwendung nach Anspruch 1, wobei der Modulator 1 bis 3 Mal nach Verabreichung des Impfstoffs und jedem Recall jeweils nur einmal täglich in einer einzigen Tagesdosis verabreicht wird.

3. Kombination von Sphingosin-1-Phosphat-Rezeptor-Modulator FTY720 mit mindestens einem Impfstoff zur sequenziellen Verwendung als immunologisches Coadjuvans bei präventiven Impfstoff-Immunisierungen, wobei der Modulator in einer Zeitspanne von 6 bis 72 Stunden nach der Verabreichung des Impfstoffs und jedem Recall verabreicht wird.

4. Kombination zur Verwendung nach Anspruch 3, wobei der mindestens eine Impfstoff für den menschlichen oder tierärztlichen Gebrauch bestimmt ist.

5. Kombination zur Verwendung nach einem der Ansprüche 3-4, wobei der mindestens eine Impfstoff ausgewählt ist aus der Gruppe, bestehend aus Impfstoff gegen Diphtherie, virale Hepatitis, Röteln, Meningitis, HPV humanes Papillomavirus, Tetanus, Poliomyelitis, Masern, Keuchhusten, Mumps, Varizellen, Tuberkulose, saisonale und pandemische Grippe, internationale Reisekrankheiten, wie Typhus, Cholera, Amöbenruhr, Malaria, Gelbfieber, Dengue, Japanische Enzephalitis, Hantavirus-Infektionen, Rift-Valley-Fieber, SARS, schweres akutes respiratorisches Syndrom, West-Nil-Virusinfektion, Chikungunyafieber, Marburger hämorrhagisches Fieber.

6. Kombination zur Verwendung nach einem der Ansprüche 3-5, wobei der Modulator 1 bis 3 Mal nach Verabreichung des Impfstoffs und jedem Recall jeweils nur einmal täglich in einer einzigen Tagesdosis verabreicht wird.

## Revendications

1. Modulateur du récepteur de la sphingosine 1-phosphate pour une utilisation comme coadjuvant immunitaire dans les immunisations par vaccins préventifs, dans laquelle ledit modulateur est FTY720 et est administré dans un laps de temps qui va de 6 à 72 heures après l'administration du vaccin et de chaque rappel.

2. Modulateur du récepteur de la sphingosine 1-phosphate selon la revendication 1, pour une utilisation selon la revendication 1, dans laquelle ledit modulateur est administré de 1 à 3 fois après l'administration du vaccin et de chaque rappel, chaque fois en une seule dose quotidienne une seule fois par jour.

3. Combinaison du modulateur du récepteur de la sphingosine 1-phosphate FTY720 avec au moins un vaccin pour l'utilisation séquentielle en tant que coadjuvant immunologique dans des immunisations par vaccins préventifs, dans laquelle ledit modulateur est administré dans un laps de temps qui va de 6 à 72 heures après l'administration du vaccin et de chaque rappel.

4. Combinaison pour une utilisation selon la revendication 3, dans laquelle ledit au moins un vaccin est destiné à un usage humain ou vétérinaire.

5. Combinaison pour une utilisation selon l'une quelconque des revendications 3-4, dans laquelle ledit au moins un vaccin est choisi dans le groupe consistant en un vaccin contre la diphtérie, l'hépatite virale, la rubéole, la méningite, le virus du papillome humain HPV, le tétanos, la poliomyélite, la rougeole, la coqueluche, les oreillons, la varicelle, la tuberculose, la grippe saisonnière et pandémique, les maladies de voyageurs internationaux parmi lesquelles la fièvre typhoïde, le choléra, l'amibiase, le paludisme, la fièvre jaune, la Dengue, l'encéphalite Japonaise, les infections à Hantavirus, la fièvre de la Vallée du Rift, le Sars, le syndrome respiratoire aigu sévère, l'infection virale du Nil Occidental, le Chikungunya, la fièvre hémorragique de Marburg.

6. Combinaison pour une utilisation selon l'une quelconque des revendications 3-5, dans laquelle ledit au moins un modulateur est administré de 1 à 3 fois après l'administration du vaccin et de chaque rappel, chaque fois en une seule dose quotidienne une seule fois par jour.
